# FASCICULE DE BREVET EUROPEEN

(11) **EP 2 825 883 B1**
(45) Date de publication et mention de la délivrance du brevet: **09.05.2018**
(21) Numéro de dépôt: 13715295.5
(22) Date de dépôt: 11.03.2013
(51) Int. Cl.: G01N 33/569, G01N 33/53, G01N 33/543

(54) **PEPTIDES D'INTERFÉRENCE ET PROCÉDÉ DE DÉTECTION DE MICROORGANISMES**
INTERFERIERENDE PEPTIDE UND VERFAHREN ZUM NACHWEIS VON MIKROORGANISMEN
INTERFERING PEPTIDES AND METHOD FOR DETECTING MICRO-ORGANISMS

(30) Priorité: 09.03.2012 FR 1252142
(43) Date de publication de la demande: 21.01.2015
(73) Titulaire: Biomérieux, 69280 Marcy l'Étoile (FR)
(72) Inventeur: BETTSWORTH, Florence, F-69380 Dommartin (FR); POTHION, Catherine, F-69005 Lyon (FR); SEIGNERES, Béatrice, F-69740 Genas (FR)
(86) Numéro de dépôt international: PCT/FR2013/050504
(87) Numéro de publication internationale: WO 2013/132198

(56) Documents cités:
- EP-A2- 0 484 787
- EP-A2- 0 582 243
- EP-A2- 0 922 958
- WO-A2-2008/027942
- WO-A2-2008/099284
- DE-A1- 4 240 980
- US-A1- 2005 271 686

## Description

La présente invention concerne le domaine du diagnostic. En particulier, elle concerne de nouveaux peptides particulièrement utiles pour éliminer les problèmes d'interférence dans le cadre de la détection *in vitro* par dosage immunologique de la présence d'un microorganisme dans un échantillon biologique, les problèmes d'interférence étant liés à l'échantillon testé.

Les méthodes de détection par dosage immunologique sont largement utilisées dans le domaine du diagnostic. Ces méthodes permettent de détecter des analytes sous forme de protéines (antigènes/anticorps), de peptides, et d'haptènes comme les stéroïdes ou les vitamines. Le dosage immunologique, également appelé procédé d'immunodosage ou test immuno-enzymatique, est un procédé largement connu de l'homme du métier impliquant des réactions immunologiques entre l'analyte à détecter et un ou des partenaire(s) de liaison à cet analyte. A titre d'exemple de tels procédés d'immunodosage, on peut citer les méthodes telles qu'ELISA, ELFA, CLIA, ECLIA, IRMA et RIA qui peuvent fonctionner selon le principe du « sandwich », ou encore selon le principe de la « compétition », et les méthodes d'immunodétection comme l'immunohistochimie, l'immunocytochimie, l'immunofluorescence, le Western-blot et le Dot-blot.

Les résultats de ces dosages immunologiques sont ensuite fournis par un Laboratoire à un Praticien qui les interprètera pour diagnostiquer un état pathologique et ensuite donner un traitement approprié au patient. Il est donc particulièrement important que ces dosages soient à la fois hautement sensibles, en ce sens qu'ils ne donnent pas de résultats faussement négatifs, et hautement spécifiques, en ce sens qu'ils ne donnent pas de résultats faussement positifs.

Une des causes altérant la sensibilité et surtout la spécificité de ces dosages est la présence d'interférences liées à l'échantillon testé. Une interférence survient chaque fois qu'une substance de l'échantillon à tester produit une réaction modifiant le signal du test, altérant ainsi la valeur correcte du résultat, par exemple en fournissant un signal qui ne peut être distingué de celui de l'analyte, donnant ainsi des faux positifs, ou en l'atténuant, donnant ainsi potentiellement des faux négatifs lorsque le signal est inférieur au seuil de détection de la trousse utilisée (Tate et Ward, 2004).

Dans le cadre des dosages immunologiques, les réactions faussement positives sont très souvent liées à des réactions non spécifiques antigène-anticorps dues à la présence, dans l'échantillon testé, de substances interférentes par rapport au dosage, telles que des anticorps, qui se lient aux partenaires de liaison utilisés pour le dosage. Par exemple, le sérum de certains patients peut contenir des anticorps humains dirigés contre des protéines animales, générés par exemple après vaccination, lesquels sont capables de réagir avec les Immunoglobulines animales entrant dans la composition de la trousse de dosage employée. Elles peuvent ainsi générer des résultats de dosage aberrants (Kricka, 1999). Les substances endogènes interférentes peuvent être présentes à la fois dans les échantillons obtenus chez les sujets sains et dans ceux obtenus chez les sujets présentant une pathologie ou ayant une infection. Le phénomène d'interférence est indépendant du statut clinique du patient.

Divers procédés pour réduire les réactions d'interférence liées à l'échantillon testé ont déjà été décrits. Ainsi, par exemple, il est connu d'utiliser des composés glucidiques, des composés protéiques, des mélanges de protéines ou des hydrolysats (EP0260903 ; US4,931,385). Il a également été décrit d'utiliser des protéines modifiées, et en particulier des protéines succinylées ou acétylées (EP0525916), ou bien des peptides de séquences en acides aminés essentiellement modifiés par rapport à la séquence native, par exemple des peptides constitués essentiellement de D-(acides aminés) (US6,153,393). Toutefois, aucun de ces procédés ne permet d'éliminer complètement la présence de faux positifs dans le dosage et l'ajout de ces substances entraînent même parfois d'autres interférences, diminuant la sensibilité du test.

D'autres réactions faussement positives peuvent être dues, non pas à l'échantillon testé, mais aux partenaires de liaison eux-mêmes utilisés pour le test, comme par exemple lorsqu'on veut détecter dans un même échantillon à la fois des antigènes et des anticorps, comme décrit dans la demande de brevet WO2008/027942. Toutefois, ces interférences liées aux partenaires de liaison sont différentes de celles liées à l'échantillon dosé et les procédés utilisés pour réduire ce type d'interférence sont différents de ceux utilisés pour réduire les interférences liées à l'échantillon dosé.

Dans la mesure où toutes les interférences ne sont pas éliminées, les Laboratoires sont parfois obligés de mettre en oeuvre des dosages alternatifs ou des mesures supplémentaires afin de vérifier le résultat de leur test diagnostic. Il est donc tout particulièrement important d'avoir des dosages dans lesquels les interférences ont été diminuées, voire éliminées, notamment quand il s'agit des faux positifs, car les patients recevraient alors une médication dont ils n'auraient pas besoin.

La Demanderesse a mis en évidence contre toute attente que, lors du dosage immunologique dans le cadre de la détection *in vitro* de la présence d'un microorganisme, il était possible, pour éliminer la détection de faux positifs dans les échantillons testés, du fait d'interférences liées à ces échantillons testés, et ce sans diminuer la sensibilité du test, d'utiliser des peptides particuliers issus de ce microorganisme, ainsi que des peptides présentant au moins 50% d'identité avec ces premiers peptides, mais issus d'un microorganisme différent du microorganisme détecté.

En effet, la Demanderesse a mis en évidence que les faux-positifs pouvaient être liés à la présence d'anticorps dirigés contre des microorganismes M1 différents du microorganisme M2 à détecter, lesquels microorganismes M1, responsables de l'interférence et dont on souhaite neutraliser la détection, présentaient une certaine identité de séquence peptidique avec celle du microorganisme M2 à détecter sur une longueur limitée, et que l'addition des peptides particuliers correspondants, appartenant aux microorganismes M1 ou M2, permettaient d'éliminer ces faux positifs sans altérer la sensibilité du test.

Ainsi, l'invention concerne l'utilisation de peptides d'interférence pour bloquer les résultats faux-positifs liés à la présence d'anticorps dirigés contre le microorganisme interférant M1, comme défini dans la revendication 1. Les peptides d'interférence sont caractérisés en ce que leur séquence est choisie parmi
(i) une séquence peptidique S1 de 7 à 12 acides aminés provenant de la séquence peptidique d'une protéine antigénique d'un microorganisme M1 interférant, et
(ii) une séquence peptidique S2 de 7 à 12 acides aminés incluse dans la séquence peptidique d'une protéine cible d'un microorganisme M2 à détecter, différent du microorganisme M1,
étant entendu que lesdites séquences S1 et S2 sont alignées l'une par rapport à l'autre, qu'elles présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences.

Un objet concerne un procédé de détection *in vitro* par immunodosage de la présence d'un microorganisme M2 dans un échantillon biologique, par la détection d'au moins un anticorps Ab_{M2} dirigé contre une protéine cible du microorganisme M2 à détecter, caractérisé en ce qu'il comprend les étapes de :
a) disposer d'au moins un partenaire de liaison audit au moins un anticorps Ab_{M2} à détecter nécessaire à l'immunodosage, ledit au moins un partenaire de liaison étant issu de ladite protéine cible contre lequel l'anticorps est dirigé ou étant la protéine cible elle-même,
b) disposer d'au moins un peptide d'interférence possédant une séquence choisie parmi
   - (i) une séquence peptidique S1 de 7 à 12 acides aminés provenant de la séquence peptidique d'une protéine antigénique d'un microorganisme M1 différent du microorganisme M2, et
   - (ii) une séquence peptidique S2 de 7 à 12 acides aminés provenant de la séquence peptidique d'une protéine cible dudit microorganisme M2 et étant incluse dans la séquence peptidique dudit au moins un partenaire de liaison,
   - étant entendu que lesdites séquences S1 et S2 sont alignées l'une par rapport à l'autre, qu'elles présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences,
c) mettre en oeuvre l'immunodosage en présence dudit au moins un peptide d'interférence et
d) détecter la présence du microorganisme M2 en mesurant le complexe formé entre l'anticorps Ab_{M2} et le ou les partenaires de liaison.

Un autre objet concerne un procédé de détection *in vitro* par immunodosage de la présence d'un microorganisme M2 dans un échantillon biologique, par la détection d'au moins une protéine cible du microorganisme M2 à détecter, caractérisé en ce qu'il comprend les étapes de :
a) disposer d'au moins un partenaire de liaison de ladite au moins une protéine cible dudit microorganisme M2, nécessaire à l'immunodosage,
b) disposer d'au moins un peptide d'interférence possédant une séquence choisie parmi
   - (i) une séquence peptidique S1 de 7 à 12 acides aminés provenant de la séquence peptidique d'une protéine antigénique d'un microorganisme M1 différent du microorganisme M2, et
   - (ii) une séquence peptidique S2 de 7 à 12 acides aminés provenant de la séquence peptidique d'une protéine cible dudit microorganisme M2 et étant incluse dans la séquence peptidique de ladite au moins une protéine cible,
   - étant entendu que lesdites séquences S1 et S2 sont alignées l'une par rapport à l'autre, qu'elles présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences,
c) mettre en oeuvre l'immunodosage en présence dudit au moins un peptide d'interférence et
d) détecter la présence du microorganisme M2 en mesurant le complexe formé entre la protéine cible et le ou les partenaires de liaison.

Un autre objet concerne un procédé d'amélioration de la spécificité d'un procédé de détection *in vitro* par immunodosage d'un analyte représentatif d'un microorganisme M2 à détecter dans un échantillon biologique, caractérisé en ce qu'il comprend l'utilisation, lors de la mise en oeuvre de l'immunodosage, d'au moins un peptide d'interférence possédant une séquence choisie parmi
(i) une séquence peptidique S1 de 7 à 12 acides aminés provenant de la séquence peptidique d'une protéine antigénique d'un microorganisme M1 différent du microorganisme M2, et
(ii) une séquence peptidique S2 de 7 à 12 acides aminés provenant de la séquence peptidique d'une protéine cible du microorganisme M2,
étant entendu que lesdites séquences S1 et S2 sont alignées l'une par rapport à l'autre, qu'elles présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences.

La Demanderesse a montré contre toute attente que la détection de faux positifs, liés à l'échantillon testé, lors de la mise en oeuvre d'un test d'immunodosage pour la détection d'un microorganisme M2 pouvait être réduite, voire éliminée, par l'utilisation de peptides particuliers, ces peptides ayant les caractéristiques suivantes :
- ils présentent une séquence peptidique S1 de 7 à 12 acides aminés provenant de la séquence peptidique d'une protéine antigénique d'un microorganisme M1 différent du microorganisme M2, ou ils présentent une séquence peptidique S2 de 7 à 12 acides aminés provenant de la séquence peptidique d'une protéine cible du microorganisme M2 à détecter,
- les séquences S1 et S2 sont alignées l'une par rapport à l'autre,
- les séquences S1 et S2 présentent au moins 50% d'identité sur leur longueur de 7 à 12 acides aminés et au moins 4 acides aminés contigus identiques ou analogues,
les séquences S1 et S2 présentent une longueur identique ou elles présentent 1 ou 2 acides aminés de différence en longueur répartis à l'une et/ou l'autre extrémité desdites séquences.

Par souci de clarté, lorsqu'on souhaitera généraliser, on appellera le peptide d'interférence utile aux fins de l'invention peptide de séquence S, que ce peptide soit de séquence S1 ou S2 et donc qu'il provienne du microorganisme M1 ou M2. On appellera alors le peptide ayant servi à l'isolement du peptide de séquence S, peptide de séquence S', sachant que ce choix tout à fait arbitraire dans la mesure où les deux peptides de séquences S1 et S2, et donc S et S', sont utilisables et utilisés en tant que peptide d'interférence selon l'invention et donc qu'on aurait pu choisir d'appeler le peptide d'interférence de séquence S1 ou S2, peptide de séquence S'.

En d'autres termes, les peptides d'interférence utiles aux fins de l'invention sont des peptides d'interférence de séquence peptidique S, de 7 à 12 acides aminés, provenant de la séquence peptidique d'une protéine antigénique d'un microorganisme M, laquelle séquence S est alignée avec une séquence peptidique différente S', de 7 à 12 acides aminés, provenant de la séquence peptidique d'une protéine cible d'un microorganisme M' différent du microorganisme M, étant entendu que lesdites séquences S et S' présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences.

Ce peptide de séquence S est particulièrement utile pour neutraliser l'échantillon vis-à-vis du microorganisme qu'on ne souhaite pas détecter (M ou M'), lequel microorganisme crée des interférences vis-à-vis du microorganisme à détecter (respectivement M' ou M). Ce dernier microorganisme peut alors être appelé microorganisme interférant.

En d'autres termes, lorsque le microorganisme cible à détecter est le microorganisme M, le peptide de séquence S, issu du microorganisme cible, sert à neutraliser le microorganisme interférant M'. En revanche, lorsque le microorganisme cible à détecter est le microorganisme M', le peptide de séquence S, qui est alors issu du microorganisme interférent, sert à neutraliser le microorganisme interférent M.

Par souci de clarté, on désignera ici le microorganisme qu'on souhaite détecter, microorganisme M2, représentant un microorganisme M' ou M, sachant que bien entendu cette appellation est arbitraire et qu'on aurait pu choisir d'appeler ce microorganisme M1.

En d'autres termes, le peptide d'interférence de séquence S de l'invention tel que défini ci-dessus est utile pour la détection d'un microorganisme M ou d'un microorganisme M'.

Les microorganismes M2 (M' ou M) dont on souhaite détecter la présence sont tout microorganisme entraînant une pathologie et dont il est nécessaire de procéder à un diagnostic fiable pour mettre en oeuvre un traitement, un suivi ou en limiter la propagation. Les microorganismes peuvent être des virus, des bactéries ou des levures, lesquelles sont bien connus de l'homme du métier.

A titre d'exemples non limitatifs de virus, on peut citer les virus des hépatites, tels que les virus des hépatites A, B, C, E et G, les virus de l'immunodéficience humaine tels que le VIH-1 et VIH-2, les virus d'Epstein-Barr (EBV), les virus de la grippe tels que H1N1 et H5N1, le cytomégalovirus (CMV), le virus de la Rougeole, le virus JC, etc.

A titre d'exemples non limitatifs de bactéries, on peut citer les Staphylocoques, tels que *Staphylococcus aureus,* les Streptocoques, tels que *Streptococcus pneumoniae,* les Bacilles.

A titre d'exemples non limitatifs de levures, on peut citer les Candida.

Par protéine cible du microorganisme M2, on entend une protéine codée par le génome dudit microorganisme et produite lors de l'infection par ce dit microorganisme, que l'on peut détecter par le procédé d'immunodosage, ou bien qui génère une réponse anticorps de la part de l'hôte infecté, également détectable par le procédé d'immunodosage. Ainsi, par exemple, dans le cadre du virus de l'hépatite C (VHC), les protéines cibles peuvent être par exemple les protéines Core, NS3, NS4 ou NS5. S'agissant du virus VIH-1, on peut citer par exemple les protéines Env (gp160, gp120, gp41), Gag (p55 et ses formes processées matures : la capside p24, la matrice p17, ...), Pol (transcriptase inverse et intégrase), Tat, Nef, Rev. A titre d'autre exemple, on peut citer les protéines Emp (extracellular matrix protein-binding protein) ou PVL (Panton-Valentine leukocydine) de la bactérie *Staphylococcus aureus*, ou les protéines « Ribosomal small subunit methyltransferase H », facteur d'élongation P, ou encore le chaperon DnaK de la bactérie *Streptococcus pneumoniae.* Bien entendu, l'homme du métier connaît parfaitement les diverses protéines cibles des microorganismes qu'il souhaite détecter.

Par microorganisme M1 (M ou M') différent du microorganisme M2 (respectivement M' ou M) à détecter, on entend tout microorganisme qui n'est pas le microorganisme M2 lui-même. Les microorganismes M1 et M2 peuvent être des microorganismes de même nature (virus, bactéries, levures) et à la même famille (par exemple le microorganisme M2 à détecter peut être le virus VHC et l'autre microorganisme est le virus VHB) ou à une autre famille (par exemple le virus à détecter est le virus VHC et l'autre microorganisme est le virus VIH-1). Les microorganismes M1 et M2 peuvent être des microorganismes de nature différente (virus/bactérie, virus/levure, bactérie/levure). Ces microorganismes sont les microorganismes que chaque patient peut rencontrer et contre lesquels il développerait une réponse anticorps.

Selon un mode de réalisation, le microorganisme M2 est un virus ou une bactérie. Selon un autre mode de réalisation, le microorganisme M2 est un virus et le microorganisme M1 est une bactérie, ou bien le microorganisme M2 est une bactérie et le microorganisme M1 est un virus

Selon un mode de réalisation, le virus est le virus de l'hépatite C, VHC, et le microorganisme différent du virus est une bactérie, notamment choisie parmi *Staphylococcus aureus, Streptococcus pneumoniae, Bacillus subtilis, Pseudomonas entomophila* et *Pseudomonas putida* (souche GB-1).

Selon un autre mode de réalisation, le virus est le virus de l'immunodéficience humaine, VIH 1, et le microorganisme différent du virus est *Mycoplasma pneumoniae.*

Par protéine antigénique d'un microorganisme M1 (ou microorganisme interférant) différent du microorganisme M2 à détecter, on entend une protéine dudit microorganisme M1 qui induit une réponse anticorps de la part de l'hôte infecté, qui est aussi le patient chez qui le prélèvement d'échantillon biologique est effectué. Là encore, l'homme du métier connaît les protéines antigéniques des microorganismes M1 qui correspondent aux protéines cibles telles que décrites ci-dessus quand on parle de détection et il pourra faire la distinction facilement entre protéines des micro organismes qui sont antigéniques ou encore appelées cibles et celles qui sont non antigéniques ou encore appelées non cibles.

L'homme du métier sait également que, comme indiqué précédemment, la différentiation entre l'expression « protéine antigénique » et « protéine cible » n'a lieu d'être que par référence au microorganisme dans le procédé de détection, à savoir à celui dont on souhaite la détection (on parle alors de protéine cible) ou à celui qu'on souhaite neutraliser (on parle alors de protéine antigénique). Il s'ensuit donc que ces deux termes sont utilisés indifféremment et sont à comprendre comme étant identiques pour le peptide d'interférence lui-même, de séquence S, sans référence à son utilisation.

Les peptides de séquence S et S', et donc de séquence S1 ou S2, selon l'invention présentent de 7 à 12 acides aminés, ce qui correspond à la longueur en acides aminés reconnue par un paratope d'un anticorps. Selon un mode de réalisation, les séquences S1 et S2 ont de 8 à 10 acides aminés.

Selon un autre mode de réalisation, les peptides d'interférence de séquence S sont choisis parmi :
- Y7E-1 de séquence SEQ ID N°1, le peptide de séquence S' étant le peptide V7E de séquence SEQ ID N°2,
- V7E de séquence SEQ ID N°2, le peptide de séquence S' étant le peptide Y7E-1 de séquence SEQ ID N°1 ,
- A8E de séquence SEQ ID N°3, le peptide de séquence S' étant le peptide E8E de séquence SEQ ID N°4,
- Y7E-2 de séquence SEQ ID N°5, le peptide de séquence S' étant le peptide E8E de séquence SEQ ID N°4,
- D8E de séquence SEQ ID N°6, le peptide de séquence S' étant le peptide E8E de séquence SEQ ID N°4,
- E8E de séquence SEQ ID N°4, le peptide de séquence S' étant le peptide A8E de séquence SEQ ID N°3 ou Y7E-2 de séquence SEQ ID N°5 ou D8E de séquence SEQ ID N°6,
- E8L-1 de séquence SEQ ID N°7, le peptide de séquence S' étant le peptide E8L-2 de séquence SEQ ID N°8, et
- E8L-2 de séquence SEQ ID N°8, le peptide de séquence S' étant le peptide E8L-1 de séquence SEQ ID N°7.

Parmi ces différents peptides, le peptide V7E du VHC de séquence SEQ ID N°2 a déjà été décrit dans les demandes de brevet EP0582243A, DE4240980A1, et EP0922980A, et le peptide AAₐ₂-QHLPYIE-BBA_{b3} a été décrit dans la demande de brevet EP0484787A. Toutefois, ces peptides n'ont été décrits qu'en tant qu'immunogène. Ils n'ont jamais été décrits en tant que peptide d'interférence utile pour supprimer les faux positifs liés à l'échantillon testé. Les autres peptides sont nouveaux et constituent un objet de l'invention.

Aussi, l'invention concerne les peptides d'interférence suivants : Y7E-1 de séquence SEQ ID N°1, A8E de séquence SEQ ID N°3, E8E de séquence SEQ ID N°4, Y7E-2 de séquence SEQ ID N°5, D8E de séquence SEQ ID N°6 et E8L-1 de séquence SEQ ID N°7.

Par « au moins 50% d'identité », on entend le fait qu'au moins la moitié des acides aminés de chaque séquence sont identiques ou analogues, étant entendu que ces deux séquences présentent au moins 4 acides aminés identiques ou analogues contigus.

De façon générale, le terme « acide aminé analogue » se réfère à un acide aminé qui, lorsqu'il remplace l'acide aminé natif dans la séquence ou lorsqu'il est absent, n'entraine aucune destruction de la réactivité antigénique de ladite séquence.

Les analogues particulièrement préférés incluent les substitutions conservatrices en nature, c'est-à-dire les substitutions qui prennent place dans une famille d'acides aminés. Il existe plusieurs classifications d'acides aminés en famille, comme bien connu de l'homme du métier. Ainsi, selon un exemple de classification, les acides aminés peuvent être divisés en 4 familles, à savoir (1) les acides aminés acides tels que l'aspartate et le glutamate, (2) les acides aminés basiques tels que la lysine, l'arginine et l'histidine, (3) les acides aminés non polaires tels que la leucine, l'isoleucine et la méthionine et (4) les acides aminés non chargés polaires tels que la glycine, l'asparagine, la glutamine, la sérine, la thréonine et la tyrosine. La phénylalanine, le tryptophane et la tyrosine sont parfois classés en acides aminés aromatiques. Par exemple, on peut prédire de façon raisonnable qu'un remplacement isolé de leucine par de l'isoleucine ou de la valine, d'un aspartate par un glutamate, d'une thréonine par une sérine, ou un remplacement conservateur similaire d'un acide aminé par un autre acide aminé ayant un rapport structurel, n'aura pas d'effet majeur sur l'activité biologique. Un autre exemple de méthode permettant de prédire l'effet sur l'activité biologique d'une substitution d'acide aminé a été décrite par Ng et Henikoff, 2001.

Les séquences S et S' (S1 et S2) sont alignées l'une par rapport à l'autre avec une longueur identique ou elles présentent 1 ou 2 acides aminés de différence répartis à l'une et/ou l'autre extrémité desdites séquences. En d'autres termes, les séquences S et S' (S1 et S2) présentent une séquence commune d'au moins 7 acides aminés, avec des acides aminés identiques ou analogues, par substitution conservatrice ou délétion comme décrit précédemment, et, lorsque leur longueur est différente, les acides aminés en plus par rapport à cette séquence commune se trouvent à une extrémité (1 ou 2 acides aminés), à l'autre (1 ou 2 acides aminés), ou aux deux (1 ou 2 acides aminés indifféremment de chaque côté. Ainsi, si on appelle X la séquence commune, Aₙ un acide aminé de différence placé à l'extrémité et n un nombre entier de 1 à 4, les alignements des peptides de l'invention peuvent être représentés comme suit :

| | | | |
|---|---|---|---|
| **X**A₁ | A₁**X** | **X**A₁A₂ | A₁A₂**X** |
| **X** | **X** | **X** | **X** |
| A₁**X**A₂ | A₁A₂**X**A₃ | A₁**X**A₂A₃ | A₁A₂**X**A₃A₄ |
| **X** | **X** | **X** | **X** |

Le nombre maximal d'acides aminés de différence est donc de 4.

L'homme du métier déterminera facilement les régions de la séquence qui peuvent tolérer un changement, sans effet majeur sur l'activité biologique, par référence aux plots Hopp/Woods et Kyte-Doolite, biens connus dans la technique.

Selon un mode de réalisation, les acides aminés de la séquence commune aux séquences S et S' (S1 et S2) remplissent au moins l'une des caractéristiques suivantes :
- ils sont identiques,
- s'ils présentent des analogues, ils présentent au plus 1 ou 2 analogues,
- ils présentent au plus 3 analogues par substitution conservatrice,
- ils présentent au plus 1 ou 2 analogues par délétion.

Selon un autre mode de réalisation, les au moins 4 acides aminés contigus remplissent au moins l'une des caractéristiques suivantes :
- ils sont identiques ou analogues par substitution conservatrice,
- quand ils présentent des analogues, ces derniers sont au plus au nombre de 1 ou 2.

Selon encore un autre mode de réalisation de l'invention, les deux séquences S et S' (S1 et S2) présentent au moins 50%, de préférence au moins 55-60%, de préférence encore au moins 70%, de préférence encore au moins 80-90% d'identité de séquence sur la longueur prédéfinie des molécules peptidiques, ainsi que toute valeur au delà de 50% et jusqu'à 100%.

Les peptides de l'invention sont particulièrement utiles pour diminuer, voire supprimer les faux positifs d'un procédé de dosage immunologique lors de la détermination de la présence d'un microorganisme M2 (M' ou M) dans un échantillon biologique susceptible de donner des faux positifs, ces faux positifs étant provoqués par la présence d'anticorps dirigés contre un microorganisme M1 (respectivement M ou M') différent du microorganisme M2 (M' ou M) à détecter (que l'on appellera Ab_{M1} ou respectivement Ab_{M} ou Ab_{M'}).

Pour mettre en oeuvre la détection de la présence du microorganisme M2 (M' ou M) à détecter, on met en oeuvre soit la détection d'au moins un anticorps dirigé contre une protéine cible du microorganisme M2 à détecter (anticorps que l'on appellera Ab_{M2} ou respectivement Ab_{M}' ou Ab_{M}), soit la détection d'au moins une protéine cible telle que décrite précédemment, soit les deux. On parle alors dans ce dernier cas d'un procédé en combinaison ou procédé combo.

Lorsque le procédé de détection *in vitro* par immunodosage de la présence d'un microorganisme M2 (M' ou M) dans un échantillon biologique comprend ou consiste en la détection d'au moins un anticorps Ab_{M2} dirigé contre une protéine cible du microorganisme M2 à détecter, ledit procédé comprend ou consiste en les étapes de :
a) disposer d'au moins un partenaire de liaison audit au moins un anticorps Ab_{M2} à détecter nécessaire à l'immunodosage, ledit au moins un partenaire de liaison étant issu de ladite protéine cible contre lequel l'anticorps est dirigé ou étant la protéine cible elle-même,
b) disposer d'au moins un peptide d'interférence tel que défini ci-dessus, étant entendu que la séquence S1 appartient au microorganisme M1 différent du microorganisme M2 à détecter et la séquence S2 est incluse dans la séquence peptidique dudit au moins un partenaire de liaison,
c) mettre en oeuvre l'immunodosage en présence dudit au moins un peptide d'interférence et
d) détecter la présence du microorganisme M2 en mesurant le complexe formé entre l'anticorps Ab_{M2} et le ou les partenaires de liaison.

Lorsque le microorganisme M2 à détecter est le microorganisme auquel appartient le peptide de séquence S (il est donc le microorganisme M), le procédé de détection *in vitro* par immunodosage de la présence d'un microorganisme M dans un échantillon biologique comprend ou consiste en la détection d'au moins un anticorps Ab_{M} dirigé contre une protéine cible du microorganisme M à détecter, ledit procédé comprend ou consiste en les étapes de :
a) disposer d'au moins un peptide d'interférence de séquence peptidique S tel que défini précédemment,
b) disposer d'au moins un partenaire de liaison audit au moins un anticorps Ab_{M} à détecter nécessaire à l'immunodosage, ledit au moins un partenaire de liaison étant issu de ladite protéine cible contre lequel l'anticorps est dirigé ou étant la protéine cible elle-même, et incluant la séquence peptidique S,
c) mettre en oeuvre l'immunodosage en présence dudit au moins un peptide d'interférence de séquence peptidique S et
d) détecter la présence du microorganisme M en mesurant le complexe formé entre l'anticorps Ab_{M} et le ou les partenaires de liaison.

Lorsque le microorganisme M2 à détecter n'est pas le microorganisme auquel appartient le peptide de séquence S, mais le microorganisme auquel appartient la séquence S' (il est donc le microorganisme M'), le procédé de détection *in vitro* par immunodosage de la présence d'un microorganisme M' dans un échantillon biologique comprend ou consiste en la détection d'au moins un anticorps Ab_{M'} dirigé contre une protéine cible du microorganisme M' à détecter, ledit procédé comprend ou consiste en les étapes de :
a) disposer d'au moins un peptide d'interférence de séquence peptidique S tel que défini précédemment,
b) disposer d'au moins un partenaire de liaison audit au moins un anticorps Ab_{M'} à détecter nécessaire à l'immunodosage, ledit au moins un partenaire de liaison étant issu de ladite protéine cible contre lequel l'anticorps est dirigé ou étant la protéine cible elle-même, et incluant la séquence peptidique S' telle que définie précédemment,
c) mettre en oeuvre l'immunodosage en présence dudit au moins un peptide d'interférence de séquence peptidique S et
d) détecter la présence du microorganisme M' en mesurant le complexe formé entre l'anticorps Ab_{M'} et le ou les partenaires de liaison.

Par l'expression « ledit au moins un partenaire de liaison étant issu de ladite protéine cible contre lequel l'anticorps est dirigé ou étant la protéine cible elle-même », on entend que le partenaire de liaison est la protéine cible telle que décrite précédemment ou bien un fragment de celle-ci capable de se lier aux anticorps de l'échantillon à doser.

Lorsque le procédé de détection *in vitro* par immunodosage de la présence d'un microorganisme M2 (M' ou M) dans un échantillon biologique comprend ou consiste en la détection d'au moins une protéine cible du microorganisme M2 à détecter, ledit procédé comprend ou consiste en les étapes de :
a) disposer d'au moins un partenaire de liaison de ladite au moins une protéine cible dudit microorganisme M2 nécessaire à l'immunodosage,
b) disposer d'au moins un peptide d'interférence tel que défini ci-dessus, étant entendu que la séquence S1 appartient au microorganisme M1 différent du microorganisme M2 à détecter et la séquence S2 est incluse dans la séquence peptidique de ladite au moins une protéine cible,
c) mettre en oeuvre l'immunodosage en présence dudit au moins un peptide d'interférence et
d) détecter la présence du microorganisme M2 en mesurant le complexe formé entre la protéine cible et le ou les partenaires de liaison.

Lorsque le microorganisme M2 à détecter est le microorganisme auquel appartient le peptide de séquence S (il est donc le microorganisme M), le procédé de détection *in vitro* par immunodosage de la présence d'un microorganisme M dans un échantillon biologique comprend ou consiste en la détection d'au moins d'au moins une protéine cible du microorganisme M à détecter, ledit procédé comprend ou consiste en les étapes de :
a) disposer d'au moins un partenaire de liaison de ladite au moins une protéine cible dudit microorganisme M nécessaire à l'immunodosage,
b) disposer d'au moins un peptide d'interférence de séquence S tel que défini ci-dessus, laquelle séquence S est incluse dans la séquence peptidique de ladite au moins une protéine cible,
c) mettre en oeuvre l'immunodosage en présence dudit au moins un peptide d'interférence de séquence S et
d) détecter la présence du microorganisme M en mesurant le complexe formé entre la protéine cible et le ou les partenaires de liaison.

Lorsque le microorganisme M2 à détecter n'est pas le microorganisme auquel appartient le peptide de séquence S, mais le microorganisme auquel appartient la séquence S' (il est donc le microorganisme M'), le procédé de détection *in vitro* par immunodosage de la présence d'un microorganisme M' dans un échantillon biologique comprend ou consiste en la détection d'au moins d'au moins une protéine cible du microorganisme M' à détecter, ledit procédé comprend ou consiste en les étapes de :
a) disposer d'au moins un partenaire de liaison de ladite au moins une protéine cible dudit microorganisme M' nécessaire à l'immunodosage,
b) disposer d'au moins un peptide d'interférence de séquence S tel que défini ci-dessus, étant entendu que la séquence S' correspondante est incluse dans la séquence peptidique de ladite au moins une protéine cible,
c) mettre en oeuvre l'immunodosage en présence dudit au moins un peptide d'interférence de séquence S et
d) détecter la présence du microorganisme M' en mesurant le complexe formé entre la protéine cible et le ou les partenaires de liaison.

A titre de partenaire de liaison à la protéine cible à rechercher, on peut citer les anticorps, les fractions d'anticorps, les nanofitines, les récepteurs à cet antigène ou toute autre protéine qui est connue pour avoir une interaction avec la protéine cible à rechercher.

Les anticorps partenaires de liaison sont par exemple soit des anticorps polyclonaux, soit des anticorps monoclonaux.

Les anticorps polyclonaux peuvent être obtenus par immunisation d'un animal avec la protéine cible ou avec la particule virale inactivée et/ou fractionnée si le microorganisme M2 (M' ou M) est un virus, ou encore avec un lysat bactérien ou un extrait de protéines bactériennes si le microorganisme M2 (M' ou M) est une bactérie, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment ladite protéine cible.

Les anticorps monoclonaux peuvent être obtenus par la technique des hybridomes dont le principe général est rappelé ci-après.

Dans un premier temps, on immunise un animal, généralement une souris avec la protéine cible d'intérêt ou avec la particule virale inactivée et/ou fractionnée si le microorganisme M2 (M' ou M) est un virus, ou encore avec un lysat bactérien ou un extrait de protéines bactériennes si le microorganisme M2 (M' ou M) est une bactérie, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de ladite protéine cible pourront être testées par exemple en ELISA, par immunotransfert (Western blot) en une ou deux dimensions, en immunofluorescence, ou à l'aide d'un biocapteur. Les anticorps monoclonaux ainsi sélectionnés sont par la suite purifiés, notamment selon la technique de chromatographie d'affinité décrite ci-dessus.

Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')₂ ainsi que les chaînes scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Que l'on détecte une protéine et/ou un anticorps, encore appelé analyte représentatif du microorganisme M2 (M' ou M) à détecter, les partenaires de liaison ont en commun qu'ils peuvent être spécifiques ou non de l'analyte à détecter. Ils sont dits spécifiques quand ils sont capables de se lier de façon exclusive ou quasi exclusive à ces analytes. Ils sont dits non spécifiques lorsque la sélectivité de liaison à ces analytes est faible et qu'ils sont alors capables de se lier à d'autres ligands, tels que d'autres protéines ou anticorps. Selon un mode de réalisation préféré, on préfère les partenaires de liaison spécifiques.

Ces partenaires de liaison spécifiques ou non des analytes recherchés dans le procédé de l'invention peuvent être utilisés comme réactif de capture, comme réactif de détection, ou comme réactifs de capture et de détection dans le cadre de l'immunodosage mis en oeuvre.

Bien entendu, le terme « immuno » dans « immunodosage » par exemple, n'est pas à considérer dans la présente demande comme indiquant strictement que le partenaire de liaison est un partenaire immunologique, tel qu'un anticorps. En effet, l'Homme du Métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (Enzyme-Linked Immunosorbent Assay) pour des dosages qui utilisent des partenaires de liaison non immunologiques, appelés plus largement en anglais « Ligand Binding Assay », que l'on pourrait traduire par « Dosage utilisant la liaison à un ligand, alors que le terme « immuno » est inclus dans l'acronyme ELISA. Par souci de clarté, la Demanderesse utilisera dans toute la demande le terme « immuno » pour tout dosage utilisant un partenaire de liaison, même quand il n'est pas un partenaire immunologique.

La mise en oeuvre de l'immunodosage est une étape largement connue de l'homme du métier qui adapte son test en fonction du microorganisme M2 (M' ou M) à détecter et des partenaires de liaison à utiliser.

Lors de ce procédé, on ajoutera un ou plusieurs peptides de l'invention et l'homme du métier adaptera les conditions du test en fonction. Le procédé d'immunodosage comprendra de préférence la mise en oeuvre d'un, deux, trois ou quatre peptides d'interférence selon l'invention.

Le ou les peptides d'interférence seront ajoutés à l'échantillon biologique à doser avant de commencer l'immunodosage ou dans l'une quelconque des étapes de réaction antigène-anticorps de l'immunodosage. Dans le cas d'un immunodosage en une étape, les peptides d'interférence seront présents ou seront ajoutés lorsque l'échantillon biologique réagit avec la phase de capture. Dans le cas d'un immunodosage en 2 étapes, les peptides d'interférence seront ajoutés lors de la capture ou lors de la révélation ou les deux. Dans tous les cas, les peptides d'interférence seront ajoutés avant l'étape de révélation (ajout du substrat et visualisation ou détection du signal).

La détection de la présence du microorganisme M2 (M' ou M) en mesurant le complexe formé entre l'analyte et les partenaires de liaison peut être mise en oeuvre par toute méthode de visualisation d'un immunodosage connu de l'homme du métier, telle que des moyens directs ou indirects.

Dans le cas de la détection directe, c'est-à-dire sans l'intermédiaire d'un marquage, on observe les réactions immunologiques par exemple par résonance plasmonique de surface ou par voltamétrie cyclique sur une électrode portant un polymère conducteur.

La détection indirecte se fait par l'intermédiaire d'un marquage, soit du partenaire de liaison dit réactif de détection, soit de la protéine cible elle-même ou d'un ou plusieurs de ses fragments.

Par marquage, on entend la fixation d'un réactif marqueur capable de générer directement ou indirectement un signal détectable. Une liste non limitative de ces réactifs marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que les Alexa ou les phycocyanines.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte le partenaire de liaison. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la Demanderesse ou à l'article de Chevalier et al., 1997.

Selon le type de marquage utilisé, l'homme du métier ajoutera des réactifs permettant la visualisation du marquage ou l'émission d'un signal détectable par un tout type d'appareil de mesure comme par exemple un spectrophomètre, un spectrofluorimètre ou encore une caméra haute définition.

L'échantillon biologique dans lequel le procédé de l'invention peut être mis en oeuvre est tout échantillon biologique susceptible de contenir un analyte représentatif d'un microorganisme (antigène, anticorps), dans lequel un immunodosage peut être mis en oeuvre. Ces échantillons sont largement connus de l'homme du métier. A titre d'exemple de tels échantillons, on peut citer les fluides biologiques tels que sérum, plasma, sang, sang total, urine, liquide céphalo-rachidien, mais aussi tissu, selles ou autre. Les échantillons biologiques proviennent de tout animal pour lequel il est nécessaire de détecter la présence d'un microorganisme pathogène, tel que mammifères, y compris l'homme, les ovins, les bovins, les caprins, les canins, poissons et espèces aviaires. La Demanderesse a montré contre toute attente que ces échantillons sont également susceptibles de contenir des anticorps Ab_{M1} dirigés contre un microorganisme différent du microorganisme M2 à détecter et que ce sont ces anticorps qui conduisent à des résultats faussement positifs du test.

L'échantillon biologique peut être traité dans une étape préalable. Par exemple, on peut utiliser des conditions acides, favorisant l'exposition des antigènes à détecter. Des détergents de type ionique ou non ionique par exemple le triton X100 ou le SDS (sodium dodecyl sulfate) ou des dérivés de poly(oxyéthylène) comme le NP40 peuvent être utilisés.

L'utilisation des peptides de l'invention est donc particulièrement utile dans tous les procédés d'immunodosage. Aussi, un autre objet de l'invention est relatif à l'utilisation d'un peptide d'interférence de séquence S tel que défini ci-dessus dans un procédé de détection *in vitro* par immunodosage d'un analyte représentatif d'un microorganisme à détecter dans un échantillon biologique.

L'utilité des peptides de l'invention résidant dans la suppression des faux positifs, améliorant ainsi la spécificité d'un test, un autre objet concerne un procédé d'amélioration de la spécificité d'un procédé de détection *in vitro* par immunodosage d'un analyte représentatif d'un microorganisme à détecter dans un échantillon biologique, caractérisé en ce qu'il comprend l'utilisation, lors de la mise en oeuvre de l'immunodosage, d'au moins un peptide d'interférence de séquence S tel que défini ci-dessus.

L'invention sera mieux comprise à l'aide des exemples suivants qui sont donnés à titre illustratif et non limitatif, ainsi qu'à l'aide des figures 1 à 4, dans lesquelles :
- la figure 1 montre l'alignement entre le peptide V7E-1 de VHC et le peptide Y7E-1 de *Staphylococcus aureus*,
- la figure 2 montre l'alignement entre le peptide E8E de VHC et le peptide A8E de *Streptococcus pneumoniae* (Fig 2A), le peptide Y7E-2 de *Bacillus subtilis* (Fig 2B) et le peptide D8E de *Pseudomonas entomophila* ou de *Pseudomonas putida* (souche GB-1) (Fig 2C),
- la figure 3 montre l'alignement entre le peptide E8L-2 de VIH-1 et le peptide E8L-1 de *Mycoplasma pneumoniae,* et
- la figure 4 montre l'alignement entre le peptide E8E de VHC et le peptide A6M de *Streptococcus pneumoniae.*

Dans les figures, « . » représente une identité d'acide aminé par rapport à l'acide aminé présent dans la même position au-dessus, et « -°» représente une délétion sur une position donnée.

### Exemple 1 : Peptides d'interférence Y7E-1 (Staphylococcus aureus) et V7E (VHC)

Afin d'améliorer la spécificité des immunodosages permettant de diagnostiquer une infection par VHC ou par *Staphylococcus aureus*, deux peptides d'interférence selon l'invention ont été définis.

Le peptide Y7E-1 a pour séquence peptidique YSPTHYVPE (SEQ ID°1), qui correspond aux acides aminés 322-330 de la protéine « Extracellular matrix protein-binding protein Emp » de *Staphylococcus aureus* (numéro d'accession Q8NXI8 de la base de données UniProtKB). Ce peptide Y7E s'aligne avec le peptide VHC V7E dont la séquence peptidique est VSPTHYVPE (SEQ ID°2) et qui correspond aux acides aminés 218-226 de la protéine NS4B du VHC (numérotation selon la séquence de référence RefSeq NCBI, le numéro d'accession est NP_751926).

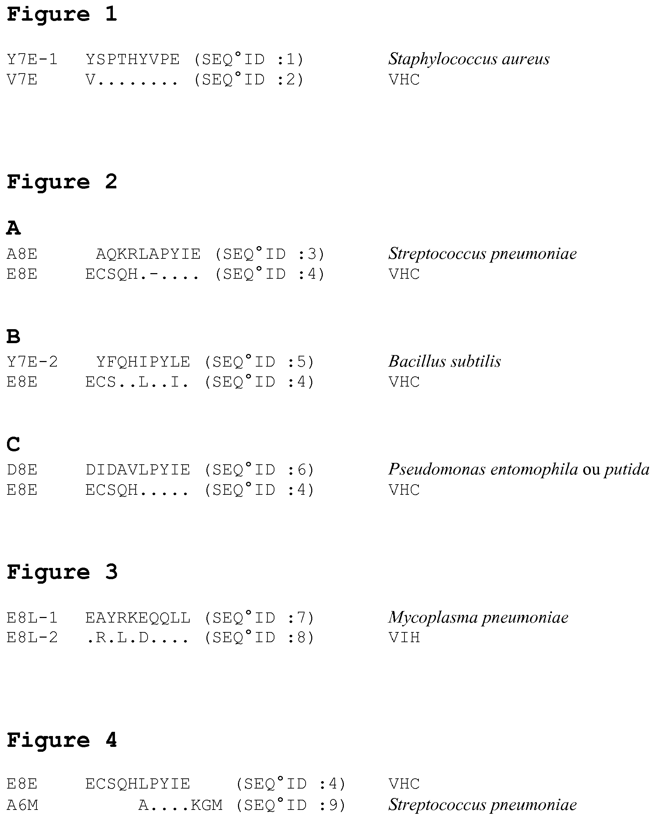

L'alignement des peptides Y7E-1 et V7E est présenté dans la Figure 1. Les peptides Y7E-1 et V7E ont une identité de 89% pour une longueur commune de 9 acides aminés.

### Exemple 2 : Peptides d'interférences E8E (VHC) et A8E (Streptococcus pneumoniae), Y7E-2 (Bacillus subtilis) et D8E (Pseudomonas entomophila ou putida souche GB-1)

Afin d'améliorer la spécificité des immunodosages permettant de diagnostiquer une infection par VHC, *Streptococcus pneumoniae, Bacillus subtilis,* ou *Pseudomonas entomophila* ou *putida* souche GB-1, plusieurs peptides d'interférence selon l'invention ont été définis.

Le peptide A8E a pour séquence peptidique AQKRLAPYIE (SEQ ID°3), qui correspond aux acides aminés 64-73 de la protéine « Ribosomal small subunit methyltransferase H » de *Streptococcus pneumoniae* (numéro d'accession C1CPL0 de la base de données UniProtKB). Ce peptide s'aligne avec le peptide VHC E8E dont la séquence peptidique est ECSQHLPYIE (SEQ ID°4) et qui correspond aux acides aminés 53-54 de la protéine NS4A du VHC (NP_751925) fusionnés aux acides aminés 1-8 de la protéine NS4B du VHC (NP_751926). L'alignement des peptides A8E et E8E est présenté dans la Figure 2A. Les peptides A8E et E8E ont une identité de 50% pour une longueur commune de 10 acides aminés.

Des peptides d'autres microorganismes que *Streptococcus pneumoniae* peuvent aussi être alignés avec le peptide VHC E8E de l'invention. Le peptide Y7E-2 a pour séquence peptidique YFQHIPYLE (SEQ ID°5), qui correspond aux acides aminés 84-92 de la protéine « Methonine-binding lipoprotein metQ » de *Bacillus subtilis* (numéro d'accession O32167 de la base de données UniProtKB). L'alignement des peptides Y7E-2 et E8E est présenté dans la Figure 2B. Les peptides Y7E-2 et E8E ont une identité de 55% pour une longueur commune de 9 acides aminés.

Le peptide D8E a pour séquence peptidique DIDAVLPYIE (SEQ ID°6), qui correspond aux acides aminés 97-106 de la protéine « Elongation factor P°» de *Pseudomonas entomophila* (numéro d'accession Q1ID35 de la base de données UniProtKB) et de *Pseudomonas putida* (souche GB-1) (numéro d'accession BOKUN5 de la base de données UniProtKB). L'alignement des peptides D8E et E8E est présenté dans la Figure 2C. Les peptides D8E et E8E ont une identité de 50% pour une longueur commune de 10 acides aminés.

### Exemple 3 : Peptides d'interférences E8L-1 (Mycoplasma pneumoniae) et E8L-2 (VIH-1)

Afin d'améliorer la spécificité des immunodosages permettant de diagnostiquer une infection par VIH-1 ou par *Mycoplasma pneumoniae,* des peptides d'interférence selon l'invention ont été définis.

Le peptide E8L-1 a pour séquence peptidique EAYRKEQQLL (SEQ ID°7), qui correspond aux acides aminés 200-209 de la protéine « tRNA (guanine-N(1)-)-methyltransferase » de *Mycoplasma pneumoniae M129* (numéro d'accession P75132 de la base de données UniProtKB). Ce peptide s'aligne avec le peptide VIH-1 E8L-2 dont la séquence peptidique est ERYLKDQQLL (SEQ ID°8) et qui correspond aux acides aminés 584-593 de la glycoprotéine d'enveloppe gp160 du VIH-1 (numérotation selon la séquence de référence RefSeq NCBI, le numéro d'accession est NP_057856). La position exacte peut varier d'une souche de VIH-1 à une autre, étant donné la grande variabilité génétique de ce virus. L'alignement des peptides E8L-1 et E8L-2 est présenté dans la Figure 3. Les peptides E8L-1 et E8L-2 ont une identité de 70% pour une longueur de 10 acides aminés.

### Exemple 4 : Amélioration de la spécificité d'un immunodosage des anticorps anti-VHC par utilisation de peptides d'interférence selon l'invention

Le diagnostic d'une infection par le virus de l'hépatite C se fait actuellement en utilisant des immunodosages qui sont des tests sérologiques immuno-enzymatiques de 3^{ème} génération et qui mettent en évidence des anticorps dirigés contre les protéines Core, NS3, et NS4, et NS5 pour certains tests. Ces anticorps anti-VHC détectés sont témoins d'une infection actuelle ou passée.

Les tests sérologiques immuno-enzymatiques permettant de mettre en évidence des anticorps anti-virus peuvent être réalisés en microplaque, de façon automatisée ou manuelle, ou encore en utilisant des automates d'immunoanalyse comme le VIDAS® (bioMérieux). Dans ce cas, toutes les étapes du test sont réalisées automatiquement par l'instrument. Les différentes techniques immuno-enzymatiques et en particulier l'ELISA sont bien connus par l'homme du métier et les grands principes ainsi que des exemples de protocole sont décrits dans le livre « The ELISA Guidebook », second édition, par John R. Crowther, éditions Humana Press (DOI : 10.1007/978-1-60327-254-4).

Le cône (SPR®) à usage unique sert à la fois de phase solide et de système de pipetage. La surface du cône est revêtue des antigènes pour la détection des anticorps dirigés contre les protéines Core, NS3 et NS4 du VHC. Dans une première étape l'échantillon est dilué, puis aspiré et refoulé à l'intérieur du cône. Les anticorps anti-VHC présents dans l'échantillon vont se fixer aux antigènes présents à l'intérieur du cône. Des étapes de lavages éliminent les composés non fixés. Au cours de la seconde étape des IgG monoclonales (souris) anti-immunoglobulines (anti-IgG) humaines sous forme Fab' conjuguées à la phosphatase alcaline recombinante sont aspirées et refoulées à l'intérieur du cône et vont se lier aux Ig humaines anti-VHC issues de l'échantillon testé, fixées sur les antigènes de la phase solide. A nouveau des étapes de lavages éliminent les composés non fixés. Lors de l'étape finale de révélation, le substrat (4-Méthylombelliferyl phosphate) est aspiré puis refoulé dans le cône ; l'enzyme du conjugué catalyse la réaction d'hydrolyse de ce substrat en un produit (4-Méthylombelliferone) dont la fluorescence émise est mesurée à 450 nm. La valeur du signal de fluorescence est proportionnelle à la concentration de l'anticorps présent dans l'échantillon. A la fin du test, les résultats sont calculés automatiquement sous forme d'un indice par l'instrument en divisant la valeur obtenue par la valeur du standard S1 ramenée à 1 (valeur mémorisée dans l'instrument). Ainsi, un échantillon dont l'indice est supérieur ou égal à 1 est considéré comme positif par le test immuno-enzymatique (présence d'anticorps anti-VHC) et un échantillon dont l'indice est inférieur à 1 est considéré comme négatif (absence d'anticorps anti-VHC). Dans les paragraphes qui suivent ce mode opératoire du dosage des anticorps-VHC sera appelé « Mode Opératoire 1 » qui correspond au dosage des anticorps anti-VHC sans peptide d'interférence.

Afin de diminuer les interférences liées à une reconnaissance non spécifique des antigènes du Mode Opératoire 1 pour la détection des anticorps anti-NS4 du fait des éléments contenus dans les échantillons testés, différents peptides d'interférence définis dans les exemples 1 et 2 ont été utilisés. Il s'agit de peptides qui appartiennent soit au virus à détecter (utilisation d'un peptide de séquence S de l'invention pour la détection d'un microorganisme M), soit à un microorganisme différent du virus à détecter (utilisation d'un peptide de séquence S de l'invention pour la détection d'un microorganisme M'). Ces peptides ont été produits par synthèse chimique selon les procédures bien connues de l'homme du métier telle que la synthèse peptidique en phase solide décrite par Merrifield, 1962 et Fields et Noble, 1990, utilisant un polymère de type polystyrène contenant 0,1-1,0 mMol amines/g de polymère. En fin de synthèse chimique, les peptides peuvent être déprotégés et clivés du polymère en présence d'un mélange d'acide trifluoroacétique-éthanedithiol-triisopropylsilane-eau (94/2.5/1/2.5 V/V/V/V) pendant environ 2 heures. Après élimination du polymère, les peptides sont extraits par précipitation dans de l'éther diéthylique à 0°C. Ils peuvent être purifiés par des techniques telles que la chromatographie liquide haute performance. La lyophilisation des fractions de purification appropriées conduit à un peptide homogène qui pourra être caractérisé par des techniques physicochimiques standard telles que la spectrométrie de masse, la chromatographie liquide haute performance, l'analyse d'acides aminés.

Plusieurs centaines d'échantillons de sérum, négatifs envers l'infection VHC et provenant des Centres de Transfusion Sanguine de Rhône-Alpes, ont été criblés afin d'identifier les échantillons qui posaient un problème d'interférence. Pour ce faire, les échantillons ont été dosés selon le Mode Opératoire 1 en utilisant l'automate VIDAS. Seuls les sérums qui étaient positifs selon le Mode Opératoire 1 (indice ≥ 1) ont été sélectionnés.

Une telle discordance entre le résultat du Mode Opératoire 1 et celui du statut négatif établi par les Centres de Transfusion Sanguine permet de sélectionner les sérums faussement positifs, posant un problème d'interférence.

Afin de montrer que l'utilisation de peptides d'interférence selon l'invention n'altére pas la sensibilité du test, on a également procédé au dosage d'un échantillon contrôle positif C1 issu d'un pool de sérum du commerce (TRINA BIOREACTIVES AG).

Le Tableau 1 présente pour 7 sérums posant un problème d'interférence et pour le contrôle positif C1, les résultats de dosages réalisés selon le « Mode Opératoire 1 » avec ou sans la présence du peptide V7E (SEQ ID°2), ou du peptide Y7E (SEQ ID°1). Ces peptides ont été ajoutés à une concentration de 5 µg/mL lors de la première étape du protocole de test immuno-enzymatique qui correspond à la dilution de l'échantillon à tester et son incubation avec les antigènes présents sur la phase solide.

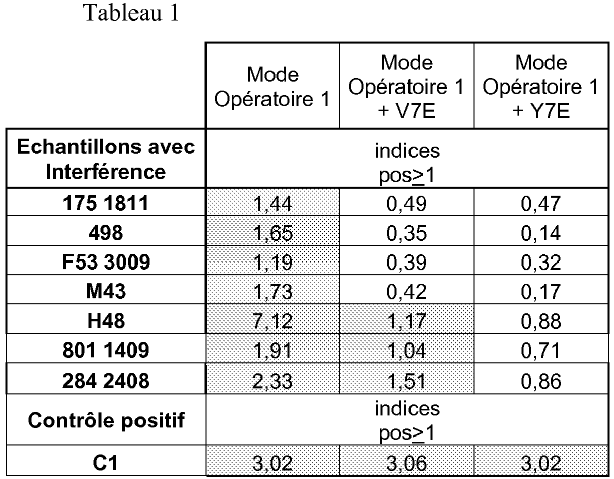

L'addition du peptide Y7E lors du dosage permet de neutraliser totalement l'interférence dans tous les sérums. Le peptide V7E permet quant à lui de neutraliser l'interférence dans 4/7 des sérums. De plus, la sensibilité du test n'est pas altérée avec l'utilisation des peptides d'interférence.

De la même manière, le Tableau 2 présente pour 2 autres sérums posant un problème d'interférence et pour le contrôle positif, les résultats de dosages réalisés selon le Mode Opératoire 1 du protocole de détection des anticorps anti-VHC, avec ou sans la présence du peptide E8E (SEQ ID°4), du peptide A8E (SEQ ID°3), ou encore du peptide A6M (SEQ ID°9). Ces peptides ont été ajoutés à une concentration de 1 µg/mL lors de la première étape du protocole de test immuno-enzymatique, qui correspond à la dilution de l'échantillon à tester, et son incubation avec les antigènes présents sur la phase solide.

Le peptide A6M dont la séquence peptidique est APYIEKGM (SEQ ID°9) est aussi un peptide de *Streptococcus pneumoniae* qui correspond aux acides aminés 69-76 de la protéine « Ribosomal small subunit methyltransferase H » de (numéro d'accession C1CPL0 de la base de données UniProtKB). Ce peptide n'est pas un peptide d'interférence selon l'invention car il ne répond pas à la définition de l'alignement telle que revendiquée (voir la figure 4). En effet, même si ce peptide présente 4 acides aminés contigus identiques au peptide E8E du VHC et est d'une longueur de 8 acides aminés, sa séquence n'est pas alignée par rapport à celle de E8E. A ce titre, ce peptide A6M est utilisé comme contrôle négatif dans l'expérience présenté dans le Tableau 2.

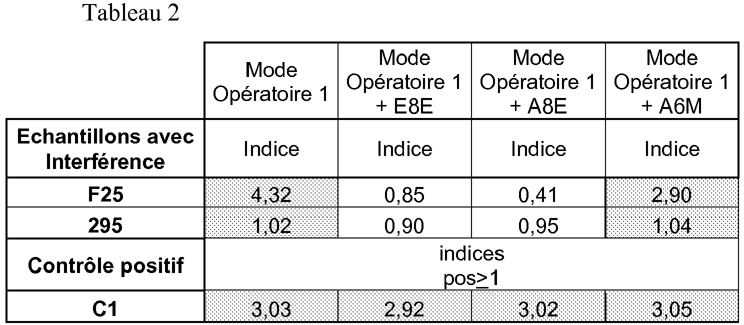

L'addition du peptide E8E ou encore du peptide A8E lors du dosage permet de neutraliser totalement l'interférence dans 2/2 des sérums. Le peptide A6M, qui n'est pas un peptide selon l'invention, ne permet pas de neutraliser l'interférence, et ce dans aucun des sérums. Là encore, la sensibilité du test n'est pas altérée.

### Références Bibliographiques

Chevalier et al., 1997, J Histochem Cytochem, 45, 481-491
Kricka LJ, 1999, Clinical Chemistry 45(7): 942-956
Fields GB, Noble RL., 1990, Int J Pept Protein Res., 35(3):161-214
Merrifield, 1962, J. Am. Chem. Soc. 85:2149
Ng et Henikoff, 2001, Genome Res 11: 863-874
Tate J et Ward G, 2004, Clin Biochem Rev, 25 : 105-120

## Revendications

1. Utilisation d'un peptide d'interférence de séquence peptidique S1 ou S2, de 7 à 12 acides aminés, la séquence S1 provenant de la séquence peptidique d'une protéine antigénique d'un microorganisme M1 interférant et la séquence peptidique S2 étant incluse dans la séquence peptidique d'une protéine cible d'un microorganisme M2 à détecter, différent du microorganisme M1, lesquelles séquences S1 et S2 sont alignées l'une par rapport à l'autre, étant entendu que lesdites séquences S1 et S2 présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences, dans un procédé de détection *in vitro* par immunodosage d'un analyte représentatif du microorganisme M2 à détecter dans un échantillon biologique, ledit analyte étant ladite protéine cible du microorganisme M2 dont est issue la séquence S2 ou un anticorps dirigé contre cette protéine, pour bloquer les résultats faux-positifs liés à la présence d'anticorps dirigés contre le microorganisme interférant M1, présents dans ledit échantillon biologique.

2. Procédé de détection *in vitro* par immunodosage de la présence d'un microorganisme M2 dans un échantillon biologique comprend ou consiste en la détection d'au moins un anticorps Ab_{M2} dirigé contre une protéine cible du microorganisme M2 à détecter, ledit procédé comprend ou consiste en les étapes de :
a) disposer d'au moins un peptide d'interférence de séquence peptidique de séquence S1 ou S2, de 7 à 12 acides aminés, la séquence peptidique S1 provenant de la séquence peptidique d'une protéine antigénique d'un microorganisme M1 interférant et la séquence peptidique S2 étant incluse dans la séquence peptidique de la protéine cible du microorganisme M2 à détecter, lesquelles séquences S1 et S2 sont alignées l'une par rapport à l'autre, étant entendu que lesdites séquences S1 et S2 présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences,
b) disposer d'au moins un partenaire de liaison audit au moins un anticorps Ab_{M2} à détecter nécessaire à l'immunodosage, ledit au moins un partenaire de liaison étant issu de ladite protéine cible contre lequel l'anticorps est dirigé ou étant la protéine cible elle-même, et incluant la séquence peptidique S2,
c) mettre en oeuvre l'immunodosage en présence dudit au moins un peptide d'interférence de séquence peptidique S1 ou S2 pour bloquer les résultats faux-positifs liés à la présence d'anticorps Ab_{M1} présents dans ledit échantillon biologique et
d) détecter la présence du microorganisme M2 en mesurant le complexe formé entre l'anticorps Ab_{M2} et le ou les partenaires de liaison.

3. Procédé de détection *in vitro* par immunodosage de la présence d'un microorganisme M2 dans un échantillon biologique comprend ou consiste en la détection d'au moins une protéine cible du microorganisme M2 à détecter, ledit procédé comprend ou consiste en les étapes de :
a) disposer d'au moins un partenaire de liaison de ladite au moins une protéine cible dudit microorganisme M2 nécessaire à l'immunodosage,
b) disposer d'au moins un peptide d'interférence de séquence peptidique S1 ou S2, de 7 à 12 acides aminés, la séquence peptidique S1 provenant de la séquence peptidique d'une protéine antigénique d'un microorganisme M1 interférant et la séquence peptidique S2 étant incluse dans la séquence peptidique de la protéine cible du microorganisme M2 à détecter, lesquelles séquences S1 et S2 sont alignées l'une par rapport à l'autre, étant entendu que lesdites séquences S1 et S2 présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences
c) mettre en oeuvre l'immunodosage en présence dudit au moins un peptide d'interférence de séquence S1 ou S2 pour bloquer les résultats faux-positifs liés à la présence d'anticorps Ab_{M1} présents dans ledit échantillon biologique et
d) détecter la présence du microorganisme M2 en mesurant le complexe formé entre la protéine cible et le ou les partenaires de liaison.

4. Procédé d'amélioration de la spécificité d'un procédé de détection *in vitro* par immunodosage d'un analyte représentatif d'un microorganisme M2 à détecter, dans un échantillon biologique, l'analyte étant une protéine cible du microorganisme M2 ou un anticorps dirigé contre cette protéine cible, **caractérisé en ce qu'**il comprend l'utilisation, lors de la mise en oeuvre de l'immunodosage, d'au moins un peptide de séquence peptidique S1 ou S2, la séquence peptidique S1 provenant de la séquence peptidique d'une protéine antigénique d'un microorganisme M1 interférant et la séquence peptidique S2 étant incluse dans la séquence peptidique de la protéine cible du microorganisme M2 à détecter, différent du microorganisme M1, lesquelles séquences S1 et S2 sont alignées l'une par rapport à l'autre, étant entendu que lesdites séquences S1 et S2 présentent au moins 50% d'identité sur leur longueur de 7 à 12 acides aminés et au moins 4 acides aminés contigus identiques ou analogues, que leur longueur est identique ou qu'elles présentent 1 ou 2 acides aminés de différence répartis à l'une et/ou l'autre extrémité desdites séquences, pour bloquer les résultats faux-positifs liés à la présence d'anticorps dirigés contre le microorganisme interférant M1, présents dans ledit échantillon biologique.

5. Utilisation ou procédé selon l'une des revendications 1 à 4, **caractérisé en ce que** les séquences S1 et S2 ont de 8 à 10 acides aminés.

6. Utilisation ou procédé selon l'une des revendications 1 à 5, **caractérisé en ce que** le microorganisme M2 à détecter est une bactérie et le microorganisme interférant M1 est un virus.

7. Utilisation ou procédé selon l'une des revendications 1 à 5, **caractérisé en ce que** le microorganisme M2 à détecter est un virus et le microorganisme interférant M1 est une bactérie.

8. Utilisation ou procédé selon l'une des revendications 6 ou 7,, **caractérisé en ce que** le virus est le virus de l'hépatite C (VHC).

9. Utilisation ou procédé selon la revendication 8, **caractérisé en ce que** le microorganisme différent du virus VHC est choisi parmi *Staphylococcus aureus*, *Streptococcus pneumoniae, Bacillus subtilis, Pseudomonas entomophila* et *Pseudomonas putida* (souche GB-1).

10. Utilisation ou procédé selon l'une quelconque des revendications précédentes, **caractérisé en ce que** les peptides de séquence S1 et S2 sont choisis parmi Y7E-1 de séquence SEQ ID N°1, V7E de séquence SEQ ID N°2, A8E de séquence SEQ ID N°3, Y7E-2 de séquence SEQ ID N°5, D8E de séquence SEQ ID N°6 et E8E de séquence SEQ ID N°4.

11. Utilisation ou procédé selon l'une quelconque des revendications 8 à 10, **caractérisé en ce que** les peptides de séquence S1 et S2 sont respectivement Y7E-1 de séquence SEQ ID N°1 et V7E de séquence SEQ ID N°2.

12. Utilisation ou procédé selon l'une quelconque des revendications 6 ou 7, **caractérisé en ce que** le virus à détecter est le virus de l'immunodéficience humaine VIH 1.

13. Utilisation ou procédé selon la revendication 12, **caractérisé en ce que** le microorganisme différent du virus à détecter est *Mycoplasma pneumoniae.*

14. Utilisation ou procédé selon la revendication 12, **caractérisé en ce que** les peptides de séquences S1 et S2 sont choisis parmi E8L-1 de séquence SEQ ID N°7 et E8L-2 de séquence SEQ ID N°8.

15. Peptide Y7E-1 de séquence SEQ ID N°1

16. Peptide A8E de séquence SEQ ID N°3.

17. Peptide E8E de séquence SEQ ID N°4.

18. Peptide Y7E-2 de séquence SEQ ID N°5.

19. Peptide D8E de séquence SEQ ID N°6.

20. Peptide E8L-1 de séquence SEQ ID N°7.

## Patentansprüche

1. Verwendung eines interferierenden Peptids einer Peptidsequenz S1 oder S2 von 7 bis 12 Aminosäuren, wobei die Sequenz S1 von der Peptidsequenz eines antigenen Proteins eines interferierenden Mikroorganismus M1 herrührt und die Peptidsequenz S2 in der Peptidsequenz eines Zielproteins eines nachzuweisenden Mikroorganismus M2, der von dem Mikroorganismus M1 verschieden ist, enthalten ist, wobei die Sequenzen S1 und S2 in Bezug zueinander ausgerichtet sind, wobei selbstverständlich ist, dass die Sequenzen S1 und S2 mindestens 50% Identität über ihre Länge von 7 bis 12 Aminosäuren und mindestens 4 identische oder analoge aufeinanderfolgende Aminosäuren aufweisen, dass ihre Länge identisch ist oder dass sie 1 oder 2 unterschiedliche Aminosäuren aufweisen, die auf das eine und/oder das andere Ende der Sequenzen verteilt sind, in einem Verfahren zum in vitro-Nachweis mittels Immunassay eines repräsentativen Analyten des nachzuweisenden Mikroorganismus M2 in einer biologischen Probe, wobei der Analyt das Zielprotein des Mikroorganismus M2, von dem die Sequenz S2 herrührt, oder ein gegen dieses Protein gerichteter Antikörper ist, um die falsch-positiven Ergebnisse in Zusammenhang mit dem Vorhandensein von gegen den interferierenden Mikroorganismus M1 gerichteten Antikörpern, die in der biologischen Probe vorhanden sind, zu blockieren.

2. Verfahren zum in vitro-Nachweis mittels Immunassay des Vorhandenseins eines Mikroorganismus M2 in einer biologischen Probe, das den Nachweis mindestens eines gegen ein Zielprotein des nachzuweisenden Mikroorganismus M2 gerichteten Antikörpers Ab_{M2} umfasst oder daraus besteht, wobei das Verfahren die folgenden Schritte umfasst oder daraus besteht:
a) Bereitstellen mindestens eines interferierenden Peptids der Peptidsequenz S1 oder S2 von 7 bis 12 Aminosäuren, wobei die Peptidsequenz S1 von der Peptidsequenz eines antigenen Proteins eines interferierenden Mikroorganismus M1 herrührt und die Peptidsequenz S2 in der Peptidsequenz des Zielproteins des nachzuweisenden Mikroorganismus M2 enthalten ist, wobei die Sequenzen S1 und S2 in Bezug zueinander ausgerichtet sind, wobei selbstverständlich ist, dass die Sequenzen S1 und S2 mindestens 50% Identität über ihre Länge von 7 bis 12 Aminosäuren und mindestens 4 identische oder analoge aufeinanderfolgende Aminosäuren aufweisen, dass ihre Länge identisch ist oder dass sie 1 oder 2 unterschiedliche Aminosäuren aufweisen, die auf das eine und/oder das andere Ende der Sequenzen verteilt sind,
b) Bereitstellen mindestens eines Bindungspartners für den mindestens einen nachzuweisenden Antikörper Ab_{M2}, der für den Immunassay benötigt wird, wobei der mindestens eine Bindungspartner von dem Zielprotein herrührt, gegen das der Antikörper gerichtet ist, oder das Zielprotein selbst ist und die Peptidsequenz S2 enthält,
c) Durchführen des Immunassays in Gegenwart des mindestens einen interferierenden Peptids der Peptidsequenz S1 oder S2, um die falsch-positiven Ergebnisse in Zusammenhang mit dem Vorhandensein von in der biologischen Probe vorhandenen Antikörpern Ab_{M1} zu blockieren, und
d) Nachweisen des Vorhandenseins des Mikroorganismus M2 durch Messen des zwischen dem Antikörper Ab_{M2} und dem oder den Bindungspartner (n) gebildeten Komplexes.

3. Verfahren zum in vitro-Nachweis mittels Immunassay des Vorhandenseins eines Mikroorganismus M2 in einer biologischen Probe, das den Nachweis mindestens eines Zielproteins des nachzuweisenden Mikroorganismus M2 umfasst oder daraus besteht, wobei das Verfahren die folgenden Schritte umfasst oder daraus besteht:
a) Bereitstellen mindestens eines Bindungspartners des mindestens einen Zielproteins des Mikroorganismus M2, der für den Immunassay benötigt wird,
b) Bereitstellen mindestens eines interferierenden Peptids der Peptidsequenz S1 oder S2 von 7 bis 12 Aminosäuren, wobei die Peptidsequenz S1 von der Peptidsequenz eines antigenen Proteins eines interferierenden Mikroorganismus M1 herrührt und die Peptidsequenz S2 in der Peptidsequenz des Zielproteins des nachzuweisenden Mikroorganismus M2 enthalten ist, wobei die Sequenzen S1 und S2 in Bezug zueinander ausgerichtet sind, wobei selbstverständlich ist, dass die Sequenzen S1 und S2 mindestens 50% Identität über ihre Länge von 7 bis 12 Aminosäuren und mindestens 4 identische oder analoge aufeinanderfolgende Aminosäuren aufweisen, dass ihre Länge identisch ist oder dass sie 1 oder 2 unterschiedliche Aminosäuren aufweisen, die auf das eine und/oder das andere Ende der Sequenzen verteilt sind,
c) Durchführen des Immunassays in Gegenwart des mindestens einen interferierenden Peptids der Sequenz S1 oder S2, um die falsch-positiven Ergebnisse in Zusammenhang mit dem Vorhandensein von in der biologischen Probe vorhandenen Antikörpern Ab_{M1} zu blockieren, und
d) Nachweisen des Vorhandenseins des Mikroorganismus M2 durch Messen des zwischen dem Zielprotein und dem oder den Bindungspartner(n) gebildeten Komplexes.

4. Verfahren zur Verbesserung der Spezifität eines Verfahrens zum in vitro-Nachweis mittels Immunassay eines für einen nachzuweisenden Mikroorganismus M2 repräsentativen Analyten in einer biologischen Probe, wobei der Analyt ein Zielprotein des Mikroorganismus M2 oder ein gegen dieses Zielprotein gerichteter Antikörper ist, **dadurch gekennzeichnet, dass** es die Verwendung mindestens eines Peptids der Peptidsequenz S1 oder S2 bei der Durchführung des Immunassays umfasst, wobei die Peptidsequenz S1 von der Peptidsequenz eines antigenen Proteins eines interferierenden Mikroorganismus M1 herrührt und die Peptidsequenz S2 in der Peptidsequenz des Zielproteins des nachzuweisenden Mikroorganismus M2, der von dem Mikroorganismus M1 verschieden ist, enthalten ist, wobei die Sequenzen S1 und S2 in Bezug zueinander ausgerichtet sind, wobei selbstverständlich ist, dass die Sequenzen S1 und S2 mindestens 50% Identität über ihre Länge von 7 bis 12 Aminosäuren und mindestens 4 identische oder analoge aufeinanderfolgende Aminosäuren aufweisen, dass ihre Länge identisch ist oder dass sie 1 oder 2 unterschiedliche Aminosäuren aufweisen, die auf das eine und/oder das andere Ende der Sequenzen verteilt sind, um die falsch-positiven Ergebnisse in Zusammenhang mit dem Vorhandensein von gegen den interferierenden Mikroorganismus M1 gerichteten Antikörper, die in der biologischen Probe vorhanden sind, zu blockieren.

5. Verwendung oder Verfahren nach einem der Ansprüche 1 bis 4, **dadurch gekennzeichnet, dass** die Sequenzen S1 und S2 8 bis 10 Aminosäuren besitzen.

6. Verwendung oder Verfahren nach einem der Ansprüche 1 bis 5, **dadurch gekennzeichnet, dass** der nachzuweisende Mikroorganismus M2 ein Bakterium und der interferierende Mikroorganismus M1 ein Virus ist.

7. Verwendung oder Verfahren nach einem der Ansprüche 1 bis 5, **dadurch gekennzeichnet, dass** der nachzuweisende Mikroorganismus M2 ein Virus und der interferierende Mikroorganismus M1 ein Bakterium ist.

8. Verwendung oder Verfahren nach einem der Ansprüche 6 oder 7, **dadurch gekennzeichnet, dass** es sich bei dem Virus um das Hepatitis C-Virus (HCV) handelt.

9. Verwendung oder Verfahren nach Anspruch 8, **dadurch gekennzeichnet, dass** der von dem HCV-Virus verschiedene Mikroorganismus aus *Staphylococcus aureus, Streptococcus pneumoniae, Bacillus subtilis, Pseudomonas entomophila* und *Pseudomonas putida* (Stamm GB-1) ausgewählt ist.

10. Verwendung oder Verfahren nach einem der vorhergehenden Ansprüche, **dadurch gekennzeichnet, dass** die Peptide der Sequenz S1 und S2 aus Y7E-1 der Sequenz SEQ ID Nr. 1, V7E der Sequenz SEQ ID Nr. 2, A8E der Sequenz SEQ ID Nr. 3, Y7E-2 der Sequenz SEQ ID Nr. 5, D8E der Sequenz SEQ ID Nr. 6 und E8E der Sequenz SEQ ID Nr. 4 ausgewählt sind.

11. Verwendung oder Verfahren nach einem der Ansprüche 8 bis 10, **dadurch gekennzeichnet, dass** es sich bei den Peptiden der Sequenz S1 und S2 um Y7E-1 der Sequenz SEQ ID Nr. 1 beziehungsweise V7E der Sequenz SEQ ID Nr. 2 handelt.

12. Verwendung oder Verfahren nach einem der Ansprüche 6 oder 7, **dadurch gekennzeichnet, dass** es sich bei dem nachzuweisenden Virus um das Humane Immundefizienzvirus HIV-1 handelt.

13. Verwendung oder Verfahren nach Anspruch 12, **dadurch gekennzeichnet, dass** der von dem nachzuweisenden Virus verschiedene Mikroorganismus *Mycoplasma pneumoniae* ist.

14. Verwendung oder Verfahren nach Anspruch 12, **dadurch gekennzeichnet, dass** die Peptide der Sequenzen S1 und S2 aus E8L-1 der Sequenz SEQ ID Nr. 7 und E8L-2 der Sequenz SEQ ID Nr. 8 ausgewählt sind.

15. Peptid Y7E-1 der Sequenz SEQ ID Nr. 1.

16. Peptid A8E der Sequenz SEQ ID Nr. 3.

17. Peptid E8E der Sequenz SEQ ID Nr. 4.

18. Peptid Y7E-2 der Sequenz SEQ ID Nr. 5.

19. Peptid D8E der Sequenz SEQ ID Nr. 6.

20. Peptid E8L-1 der Sequenz SEQ ID Nr. 7.

## Claims

1. Use of an interfering peptide having peptide sequence S1 or S2, of 7 to 12 amino acids, wherein sequence S1 originates from the peptide sequence of an antigenic protein of a interfering microorganism M1 and peptide sequence S2 is included in the peptide sequence of a target protein of a microorganism M2 to be detected and being different from microorganism M1, said sequences S1 and S2 being aligned with respect to one another, it being understood that said sequences S1 and S2 exhibit at least 50% identity over their length of 7 to 12 amino acids and at least 4 identical or analogous contiguous amino acids, that their length is identical or that they exhibit a difference of 1 or 2 amino acids distributed at one and/or the other end of said sequences, in a method for the *in vitro* immunoassay-based detection of an analyte representative of a microorganism M2 to be detected in a biological sample, wherein said analyte is the target protein of microorganism M2 from which sequence S2 comes from, or an antibody directed against this protein, to block the false-positive results linked to the presence of antibodies directed against the interfering microorganism M1, present in the biological sample.

2. A method for the *in vitro* immunoassay-based detection of the presence of a microorganism M2 in a biological sample comprises or consists of the detection of at least one antibody Ab_{M2} directed against a target protein of the microorganism M2 to be detected, said method comprises or consists in the steps of:
a) providing at least one interfering peptide having peptide sequence S1 or S2, of 7 to 12 amino acids, wherein sequence S1 originates from the peptide sequence of an antigenic protein of an interfering microorganism M1 and peptide sequence 52 is included in the peptide sequence of the target protein of microorganism M2 to be detected, said sequences S1 and S2 being aligned with respect to one another, it being understood that said sequences S1 and S2 exhibit at least 50% identity over their length of 7 to 12 amino acids and at least 4 identical or analogous contiguous amino acids, that their length is identical or that they exhibit a difference of 1 or 2 amino acids distributed at one and/or the other end of said sequences,
b) providing at least one binding partner for said at least one antibody Ab_{M2} to be detected, required for the immunoassay, wherein said at least one binding partner is derived from said target protein against which the antibody is directed or is the target protein itself, and including the peptide sequence S2,
c) carrying out the immunoassay in the presence of said at least one interfering peptide having peptide sequence S1 or S2 to block the false-positive results linked to the presence of Ab_{M1} antibodies present in the biological sample, and
d) detecting the presence of the microorganism M2 by measuring the complex formed between the antibody Ab_{M2} and the binding partner(s).

3. A method for the *in vitro* immunoassay-based detection of the presence of a microorganism M2 in a biological sample comprises or consists of the detection of at least one target protein of the microorganism M2 to be detected, said method comprising or consisting of the steps of:
a) providing at least one binding partner for said at least one target protein of said microorganism M2, required for the immunoassay,
b) providing at least one interfering peptide having peptide sequence S1 or S2, having 7 to 12 amino acids, wherein peptide sequence S1 originating from the peptide sequence of an antigenic protein of an interfering microorganism M1 and peptide sequence S2 is included in the peptide sequence of the target protein of microorganism M2 to be detected, said sequences S1 and S2 being aligned with respect to one another, it being understood that said sequences S1 and S2 exhibit at least 50% identity over their length of 7 to 12 amino acids and at least 4 identical or analogous contiguous amino acids, that their length is identical or that they exhibit a difference of 1 or 2 amino acids distributed at one and/or the other end of said sequences,
c) carrying out the immunoassay in the presence of said at least one interfering peptide having sequence S1 or S2 to block the false-positive results linked to the presence of Ab_{M1} antibodies present in the biological sample, and
d) detecting the presence of the microorganism M2 by measuring the complex formed between the target protein and the binding partner(s).

4. A method for improving the specificity of a method for the *in vitro* immunoassay-based detection of an analyte representative of a microorganism M2 to be detected, in a biological sample, wherein said analyte is a target protein of microorganism M2 or an antibody directed against this target protein, **characterized in that** it comprises the use, during the implementation of the immunoassay, of at least one peptide having peptide sequence S1 or S2, wherein peptide sequence S1 originates from the peptide sequence of an antigenic protein of a interfering microorganism M1 and peptide sequence S2 is included in the peptide sequence of the target protein of microorganism M2 to be detected and being different from microorganism M1, said sequences S1 and S2 being aligned with respect to one another, it being understood that said sequences S1 and S2 exhibit at least 50% identity over their length of 7 to 12 amino acids and at least 4 identical or analogous contiguous amino acids, that their length is identical or that they exhibit a difference of 1 or 2 amino acids distributed at one and/or the other end of said sequences, to block the false-positive results linked to the presence of antibodies directed against the interfering microorganism M1 present in said biological sample.

5. Use or method as claimed in any of claims 1 to 4, **characterized in that** sequences S1 and S2 have 8 to 10 amino acids.

6. Use or method as claimed in any of claims 1 to 5, **characterized in that** the microorganism M2 to be detected is a bacterium and the interfering microorganism M1 is a virus.

7. Use or method as claimed in any of claims 1 to 5, **characterized in that** the microorganism M2 to be detected is a virus and the interfering microorganism M1 is a bacterium.

8. Use or method as claimed in claim 6 or 7, **characterized in that** the virus is the hepatitis C virus (HCV).

9. Use or method as claimed in claim 8, **characterized in that** the microorganism different from the HCV virus is chosen from *Staphylococcus aureus*, *Streptococcus pneumoniae, Bacillus subtilis, Pseudomonas entomophila* and *Pseudomonas putida* (GB-1 strain).

10. Use or method as claimed in any of preceding claims, **characterized in that** the peptides having sequence S1 or S2 are chosen from Y7E-1 having the sequence of SEQ ID N°1, V7E having the sequence of SEQ ID N°2, A8E having the sequence of SEQ ID N°3, Y7E-2 having the sequence of SEQ ID N°5, D8E having the sequence of SEQ ID N°6 and E8E having the sequence of SEQ ID N°4.

11. Use or method as claimed in any of claims 8 to 10, **characterized in that** peptides having sequence S1 and S2 are respectively Y7E-1 of sequence SEQ ID N°1 and V7E of sequence SEQ ID N°2.

12. Use or method as claimed in any of claims 6 or 7, **characterized in that** the virus to be detected is the human immunodeficiency virus HIV-1.

13. Use or method as claimed in claim 12, **characterized in that** the microorganism different from the virus to be detected is *Mycoplasma pneumoniae.*

14. Use or method as claimed in claim 12, **characterized in that** the peptides having sequence S1 or S2 are chosen among E8L-1 having sequence SEQ ID N°7 and E8L-2 having sequence SEQ ID N°8.

15. Peptide Y7E-1 having the sequence of SEQ ID N°1.

16. Peptide A8E having the sequence of SEQ ID N°3.

17. Peptide E8E having the sequence of SEQ ID N°4.

18. Peptide Y7E-2 having the sequence of SEQ ID N°5.

19. Peptide D8E having the sequence of SEQ ID N°6.

20. Peptide E8L-1 having the sequence of SEQ ID N°7.


### SEQUENCE LISTING

<110> bioMérieux
<120> Peptides d'interférence et procédé de détection de microorganismes
<130> CSPECIF
<150> FR12 52142
   <151> 2012-03-09
<160> 9
<170> PatentIn version 3.5
<210> 1
   <211> 9
   <212> PRT
   <213> staphylococcus
<400> 1
   Tyr Ser Pro Thr His Tyr Val Pro Glu
   1               5
<210> 2
   <211> 9
   <212> PRT
   <213> VHC
<400> 2
   Val Ser Pro Thr His Tyr Val Pro Glu
   1               5
<210> 3
   <211> 10
   <212> PRT
   <213> Streptoccus
<400> 3
   Ala Gln Lys Arg Leu Ala Pro Tyr Ile Glu
   1               5                   10
<210> 4
   <211> 10
   <212> PRT
   <213> VHC
<400> 4
   Glu Cys Ser Gln His Leu Pro Tyr Ile Glu
   1 5 10
<210> 5
   <211> 9
   <212> PRT
   <213> Bacillus
<400> 5
   Tyr Phe Gln His Ile Pro Tyr Leu Glu
   1 5
<210> 6
   <211> 10
   <212> PRT
   <213> Pseudomonas
<400> 6
   Asp Ile Asp Ala Val Leu Pro Tyr Ile Glu
   1 5 10
<210> 7
   <211> 10
   <212> PRT
   <213> Mycoplasma
<400> 7
   Glu Ala Tyr Arg Lys Glu Gln Gln Leu Leu
   1 5 10
<210> 8
   <211> 10
   <212> PRT
   <213> VIH
<400> 8
   Glu Arg Tyr Leu Lys Asp Gln Gln Leu Leu
   1 5 10
<210> 9
   <211> 8
   <212> PRT
   <213> Streptococcus
<400> 9
   Ala Pro Tyr Ile Glu Lys Gly Met
   1               5